# EUROPEAN PATENT SPECIFICATION

(11) **EP 3 747 029 B1**
(45) Date of publication and mention of the grant of the patent: **28.02.2024**
(21) Application number: 19747206.1
(22) Date of filing: 31.01.2019
(51) Int. Cl.: A61K 35/33, A61K 35/34, A61L 15/26, A61L 15/32, A61L 15/42, H01B 1/12, C12N 15/00, C12N 5/077, C12N 5/00, H01B 1/24

(54) **METHOD AND APPARATUS FOR STIMULATION OF CELLS FOR TISSUE REPAIR**
VERFAHREN UND VORRICHTUNG ZUR STIMULATION VON ZELLEN ZUR GEWEBEREPARATUR
PROCÉDÉ ET APPAREIL DE STIMULATION DE CELLULES POUR LA RÉPARATION DE TISSUS

(30) Priority: 31.01.2018 US 201862624456 P
(43) Date of publication of application: 09.12.2020
(73) Proprietor: Illinois Institute Of Technology, Chicago IL 60616 (US)
(72) Inventor: WANG, Rong, Hinsdale, IL 60521 (US); CHI, Naiwei, Woodridge, IL 60517 (US)
(74) Representative: Rentsch Partner AG
(86) International application number: PCT/US2019/015990
(87) International publication number: WO 2019/152610

(56) References cited:
- WO-A1-2007/115388
- US-A1- 2010 144 004
- US-A1- 2011 039 089
- US-A1- 2012 121 712
- YU HONGSHENG ET AL: "Mechanically and Electrically Enhanced CNT-Collagen Hydrogels As Potential Scaffolds for Engineered Cardiac Constructs", ACS BIOMATERIALS SCIENCE & ENGINEERING, vol. 3, no. 11, 13 November 2017 (2017-11-13), pages 3017-3021, XP055839390, US ISSN: 2373-9878, DOI: 10.1021/acsbiomaterials.6b00620
- CHEN CHI-SHUO ET AL: "Human stem cell neuronal differentiation on silk carbon nanotube composite", NANOSCALE RESEARCH LETTERS, vol. 7, no. 1, 1 December 2012 (2012-12-01), XP055839236, US ISSN: 1931-7573, DOI: 10.1186/1556-276X-7-126 Retrieved from the Internet: URL:https://link.springer.com/content/pdf/ 10.1186/1556-276X-7-126.pdf>
- PAN HUI ET AL: "Significantly Reinforced Composite Fibers Electrospun from Silk Fibroin/Carbon Nanotube Aqueous Solutions", BIOMACROMOLECULES, vol. 13, no. 9, 10 September 2012 (2012-09-10), pages 2859-2867, XP055839239, US ISSN: 1525-7797, DOI: 10.1021/bm300877d
- CHI-SHUO CHEN ET AL: "Silk-carbon nanotube composite for stem cell neuronal differentiation", NANOELECTRONICS CONFERENCE (INEC), 2011 IEEE 4TH INTERNATIONAL, IEEE, 21 June 2011 (2011-06-21), pages 1-2, XP032035686, DOI: 10.1109/INEC.2011.5991795 ISBN: 978-1-4577-0379-9
- SRIDHARAN I ET AL: "Adapting collagen/CNT matrix in directing hESC differentiation", BIOCHEMICAL AND BIOPHYSICAL RESEARCH COMMUNICATIONS, ELSEVIER, AMSTERDAM NL, vol. 381, no. 4, 17 April 2009 (2009-04-17), pages 508-512, XP026031250, ISSN: 0006-291X, DOI: 10.1016/J.BBRC.2009.02.072 [retrieved on 2009-02-20]
- KIM TAEYOUNG ET AL: "Effect of CNT on collagen fiber structure, stiffness assembly kinetics and stem cell differentiation", MATERIALS SCIENCE AND ENGINEERING C, vol. 49, 7 January 2015 (2015-01-07), pages 281-289, XP029139901, ISSN: 0928-4931, DOI: 10.1016/J.MSEC.2015.01.014
- CHI NAIWEI ET AL: "Electrospun protein-CNT composite fibers and the application in fibroblast stimulation", BIOCHEMICAL AND BIOPHYSICAL RESEARCH COMMUNICATIONS, vol. 504, no. 1, 1 September 2018 (2018-09-01), pages 211-217, XP055839389, Amsterdam NL ISSN: 0006-291X, DOI: 10.1016/j.bbrc.2018.08.157

## Description

### BACKGROUND OF THE INVENTION

This invention relates generally to tissue repair and wound healing and, more particularly, to cell therapy treatment for tissue repair.

For chronic wound care, local treatments are directed toward reducing pain and itching, minimizing infection and bleeding from the wound. The treatments don't promise to heal the wound. Adult stem cells, especially mesenchymal stem cells, have been shown to play an important role in wound healing. However, isolation, expansion and differentiation of these cells require complicated controls and are time demanding, expensive and subject to strict and increasing regulatory demands. Alternatively, autologous fibroblast cells from patients are easily accessible and abundant. However the molecular and cellular abnormality of patients' fibroblast cells, such as cells in diabetes patients or in patients with connective tissue disorders, may become an obstacle of their successful application.

Wound healing is a complex process, which is typically initiated by hemostasis followed by inflammation, tissue growth and tissue remodeling. Inflammation and tissue growth are two critical steps that ensure a rapid recovery. In diabetic patients, for example, inflammation process is prolonged due to hyperglycemia. The elevated level of blood sugar results in a higher level of matrix metalloproteinases (MMPs) in the tissue. While MMPs are essential for cleaning wound bed and rapid turnover of growth factors, receptors, and newly formed extra-cellular matrix proteins, the elevated MMP level in diabetes patients accelerates the degradation of newly synthesized matrix proteins and disrupt their proper deposition and maturation, leading to chronic wounds.

MMPs are synthesized by fibroblast cells, which are also the main source of extracellular matrix (ECM) proteins that are essential to the wound healing process. Collagen is the most abundant protein in ECM. Collagen type I (COLI) and collagen type III (COLIII) are the predominant types of collagen in connective tissues. Typically, fibrillar COLI offers high tensile strength to tissues, whereas COLIII is commonly found alongside COLI in tissues that require increased flexibility and distension. In normal skin tissues, the COLI/COLIII ratio is around 2.3-2.5. During the tissue growth process in wound healing, increasing amount of COLIII is synthesized by fibroblast cells to form granulation tissues to cover up the wound area after it is cleaned by MMPs. In the skin tissues of diabetes patients, however, the COLI/COLIII ratio is much higher (about 5). Thus, a significantly higher amount of COLIII must be synthesized to overcome the high COLI/COLIII ratio in order to form granulation tissues for wound healing. Nevertheless, the elevated MMP level in diabetes patients renders the newly synthesized proteins rapidly degraded, attributing to the formation of chronic wounds.
Tissue engineering compositions and/or methods are disclosed in Yu Hongsheng et al, ACS Biomaterials Science & Engineering 2017, 3, 3017-3021; Chen Chi-Shuo et al. Nanoscale Research Letters 2012, 7, 1; Pan Hui et al. Biomacromolecules 2012, 13, 2859-2867; Chen Chi-Sho et al. Nanoelectronics conference (INEC) 2011 IEEE 4th international, IEEE, 21 June 2011, p. 1-2; Sridharan I et al. Biochemical and Biophysical Research Communications 2009, 381, 508-512; Kim Taeyoung et al Materials Science and Engineering C, 2015, 49, 281-289. Tissue engineered skin substitutes have been developed to overcome the deficiency of growth factors and cytokines, and create an environment similar to that of a normal wound healing. Concerns over infection, antigenicity and hypoglycemia undermine their usage. There is a continuing need to improve tissue regeneration and wound healing.

### SUMMARY OF THE INVENTION

The invention is set out in the appended claims. Embodiments of this invention provide for regulating the properties of a matrix that supports cell growth, and stimulating cells through the matrix. The stimulation induces a dramatic augmentation of total protein production and a renewed protein expression profile that is favored by tissue regeneration and wound healing. The stimulation results in accelerated wound healing in a model system.

The invention further includes methods and compositions for improving collagen production and/or ratios of collagen types. Such manipulation of collagen production is useful for tissue regeneration and wound healing.

The invention includes cell therapy compositions/structures and treatments, such as, for chronic wounds of diabetes patients. Instead of relying on cells from matching donors, fibroblast cells of a diabetes patient can be extracted and "corrected", then injected back to the patient at the wound area for treatment without the concern of immune-rejection. Additionally or alternatively, the free-standing biocompatible matrix with "corrected" cells can be applied or implanted as a patch. In this case, patients can undergo a physical therapy to restimulate the cells on the matrix periodically when needed. In addition to treating chronic wound of diabetes patients, the method/material has applicability for connective tissue repairs, such as pelvic floor connective tissue repair for pelvic organ prolapse treatment and surgical incision hernia repair, as well as collagen enrichment for cosmetic or health purposes.

The invention includes a cell therapy structure, including a matrix of fibers including a proteinaceous material and an electrically conductive material. Embodiments of this invention incorporate free-standing, unidirectionally aligned silk-carbon nanotube (CNT) fibers, such as prepared by electrospinning (E-spinning) techniques. The silk protein fibers incorporate a low dose of oxidized single-wall carbon nanotubes. These fibers are sufficiently flexible, tough, stable and biocompatible for effortless handling and for adequate support of cell development. These fibers are also biodegradable and electrically conductive.

The application further includes a method of tissue engineering. The method includes seeding a cell culture on a matrix of electrically conductive protein fibers; applying an electric field to the matrix to stimulate the cell culture. The application of an electric field can, for example, stimulate fibroblast cells on the silk-CNT fiber matrix to supply a great amount of collagen with low COLI/COLIII ratio, which is favorable in forming granulation tissue during wound healing. The electrical stimulation also suppresses the cell synthesis of MMPs. This provides a proper balance between collagen synthesis and degradation to allow the granulation tissue building up at the wound area, without the overexpression of MMPs in diabetic fibroblast cells and results in acceleration of matrix protein degradation leading to chronic wounds.

Other objects and advantages will be apparent to those skilled in the art from the following detailed description taken in conjunction with the appended claims and drawings.

### BRIEF DESCRIPTION OF THE DRAWINGS

Fig. 1 representatively illustrates a cell therapy apparatus, according to one embodiment of this invention.
Fig. 2 illustrates a method of cell therapy, according to one embodiment of this invention.
Fig. 3 illustrates a method of cell therapy, according to another embodiment of this invention.
Fig. 4 shows a comparison of COLI and COLIII gene expression profiles of non-diabetic dermal fibroblasts (NDF) and diabetic dermal fibroblasts (DDF) cells grown on plastic, silk fibers and silk-CNT (0.05%) fibers, with a) showing the relative COLI and COLIII expression levels of NDF and DDF cells after 5 days culture on various matrices, and b) showing a comparison of COLI/COLIII ratio in NDF and DDF cells after 5 days culture on various matrices, with the ratios derived from mean values in (a).
Fig. 5 summarizes effects of electrical stimulation on cell proliferation and viability, with a) showing stimulation time dependence of DDF cell proliferation profiles, and b) showing stimulation time dependence of cell viability for NDF and DDF cells on silk-CNT (0.05%) fibers at 24 h post-plating.
Fig. 6 summarizes COLI and COLIII gene expression profiles of NDF and DDF cells grown on plastic (a), silk fibers (b) and silk-CNT (0.05%) fibers (c) for 12 h with or without 6 h electrical stimulation.
Fig. 7 summarizes wound healing experiment with a nude mouse having 4 mm × 4 mm skin excisional wounds of 4 mm deep were created at the dorsum of the mouse, with a) being a comparison of wound healing over time for wounds with the application of electrically stimulated DDF cells (lower panel) and without any treatment (upper panel), b) showing a decrease of wound area over time for the wound with no treatment and the wound treated by electrically stimulated cells, c) showing a decrease of wound area over time for the wounds treated by unstimulated cells and electrically stimulated cells.
Fig. 8 shows variation of mechanical properties with CNT percentage in E-spun collagen and silk fibers, with a) summarizing ultimate strain, b) summarizing ultimate tensile strength, c) summarizing Young's modulus, and d) being optical and AFM images of well aligned silk-CNT-0.05% fibers and of less aligned silk-CNT-0.5% fibers (the arrow marks the surface defect that was frequently observed in fibers at a high CNT percentage).
Fig. 9 shows E-spun fiber alignment and conductivity, with a) showing variation of fiber alignment, b) showing protein solution viscosity, c) showing optical image illustrating the freestanding silk-CNT fibers aligned in parallel between two gold electrodes for conductivity measurement and current-voltage curves, and d) summarizing CNT percentages.
Fig. 10 shows fibroblast polarization on various E-spun fibers, with a) being optical images illustrating cell polarization overtime on various substrates, and b) summarizing cell length-to-width ratio derived from images of cells grown on various substrates at different time point.

### DESCRIPTION OF THE INVENTION

The present invention provides a method and apparatus for cell and/or tissue engineering, such as for use in wound healing. Embodiments of this invention include a support matrix on which cells, such as fibroblast or muscle cells, preferably from the patient, are supported. In embodiments of this invention, the cells on the matrix are stimulated to produce tissue components that increase wound healing.

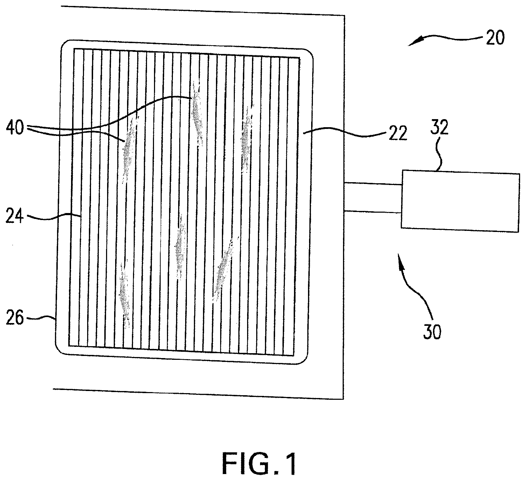

Fig. 1 generally illustrates a cell therapy apparatus 20 according to embodiments of this invention. The apparatus 20 includes a matrix 22 which is formed of fibers 24. The fiber matrix 22 is supported on any suitable support substrate 26, such as a polymer or silicone substrate, for example, a silicone gel substrate. The fibers 24 are electrically conductive, able to receive and conduct current from a suitable electrical stimulator 30 having power source 32 in electric supply combination with the fiber matrix 22. In embodiments of the invention, the fibers 24 are arranged in parallel to promote cell polarization and current conductance across the matrix 22 between longitudinal ends of the fibers 24.

The fibers used in the experiments below were approximately 1 µm thick, mimicking the dimension of native collagen fibers in extracellular matrix and about 1 cm long. As elongated fibroblast cells are about 30-50 µm long, 1-cm silk-CNT fibers are sufficiently long to stimulate the cells. The thickness and length can be controlled by adjusting the electrospinning conditions. As will be appreciated, variations in size, shape, and configuration can vary, depending on need.

In embodiments of this invention, the fibers are formed (e.g., spun) from a combination of a proteinaceous material and an electrically conductive material. For example, the fibers are spun from a combination of the electrically conductive material, such as carbon nanotubes, and a fibroin material or a collagen material. Matrix fibers are composite silk-carbon nanotube (CNT) fibers such as electrospun silk-CNT fibers, which are free-standing, well-aligned, electrically conductive, stable, biocompatible and biodegradable. These fibers are easy to prepare and easy to manage. Current study suggests that the low dose usage of CNT in silk composite fibers is safe. Thus they can be used both *in vitro* and *in vivo.* Individual fibers of embodiments of this invention include about 0.05% to about 0.5% by weight carbon nanotubes, and more preferably about 0.05% to about 0.25% by weight carbon nanotubes.

Silk materials, such as spider silk proteins or silkworm silk fibroin, are useful for preparing aligned silk-CNT matrix for mediated fibroblast cell stimulation according to this invention. These fibrous matrices can be applied in a similar way to stimulate fibroblast cells for increased collagen production. For example, silk-CNT matrices can be used to direct and accelerate stem cell differentiation toward neuronal cells. The application of electrical stimulation according to this invention will provide additional cues that may further accelerate neural differentiation of stem cells.

Embodiments of this invention include restoring the functionality of patients' cells *in vitro* via matrix mediated electrical stimulation and resupplying the transformed cells to patients to revive tissue function. The use of autologous cells to treat, for example, connective tissue wounds or disorders is relatively safe and simple, effectively avoiding complications in other approaches, such as immunological rejection of cells derived from other individuals, complicated control of isolation, expansion and differentiation conditions of stem cells, heterogeneity between cells of the same type (e.g., fibroblast cells) but from different origin (e.g., dermal vs. pelvic floor). Fibroblast cells, for example, are relatively abundant and easy to harvest. It is expected to be an economically favorable and clinically relevant alternative of reconstructive surgery or stem cell therapy.

Fig. 1 shows a representative cell culture 40 supported on the finer matrix 22. Fibroblast cells are illustrated, and referred to herein for explanation. Other types of cells can be used as well, such as smooth muscle cells. In embodiments of this invention, cell stimulation can be achieved by applying an electric field to the fiber matrix 22 that supports a cell culture. The electrical stimulation regulates or improves cell function, such as improving protein productivity of the cell culture 40, and alternatively or additionally can promote cell proliferation, contraction, migration, and/or activation/differentiation. The aligned fibers and/or electric field generally also has an effect of polarizing the fibroblast cells in the direction of the alignment, as illustrated.

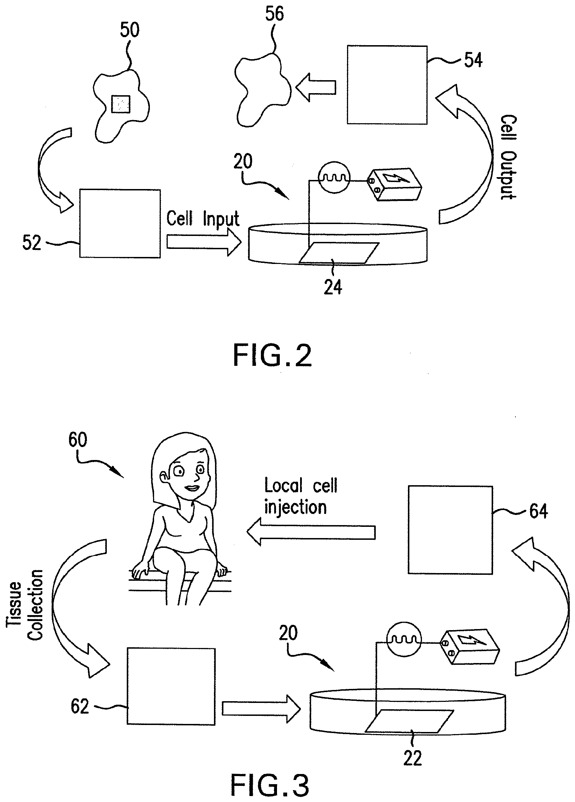

Fig. 2 illustrates a method of use of a cell therapy apparatus 20 according to one embodiment of this invention. In Fig. 2, fibroblasts 52 from wounded skin 50 serve as the cell input on the fiber matrix 22 of apparatus 20. Electrical stimulation is applied to the cells to obtain a cell output, such as transformed fibroblast cells 54 ready to be used in tissue repair of the skin 50, resulting in healed skin 56.

Fig. 3 illustrates a method of use of a cell therapy apparatus 20 according to another embodiment of this invention. Tissue is collected from the patient 60, for fibroblast 62 extraction. As in Fig. 2, fibroblasts 62 serve as the cell input on the fiber matrix 22 of apparatus 20. Electrical stimulation is applied to the cells to obtain a cell output, such as transformed fibroblast cells 64, which are gathered from the matrix 22 for localized injection back to the patient 60. In addition to localized injection, the fiber matrix can be applied to the patient before or after stimulation, for example, as a bandage or implant, depending on treatment need. For example, the matrix can be used as a physiotherapy to continually or periodically stimulate the cells as needed by applying stimulation after application of the matrix to the patient.

As discussed in the examples below, stimulated diabetic dermal fibroblast cells renewed the profile of matrix protein expression and accelerated wound healing in an animal model. The same strategy can be applied to transform fibroblast cells from pelvic organ prolapse (POP) patients *in vitro* and revive their matrix protein productivity. Replenishing transformed autologous cells to POP patients can not only restore the matrix protein expression profile, but also recondition the microenvironment of smooth muscle cells (SMCs) for improved elastin synthesis, better collagen, elastin and SMC interdigitation and consequently, improved elasticity and toughness of the connective tissues. The effect can be long-lasting as not only the cells are transformed, the matrix is also reconditioned.

Vaginal wall fibroblast cells are relatively abundant, easy to harvest, culture and expansion *in vitro,* making them an ideal cell source for regenerative medicine. The use of autologous vaginal wall fibroblast cells is relatively safe, the matrix mediated cell stimulation/transformation is efficient, and the local cell administration is straightforward, making the procedure affordable for most patients. In addition to POP tissue repair, the non-invasive, local cell administration of embodiments of this invention can also be applied to renew the elasticity of vaginal wall tissues for older women to improve their quality of life. The invention can also be applied to replenishing and stimulating smooth muscle cells or a mixture of fibroblast and smooth muscle cells to achieve further improvement of the mechanical strength of POP tissues.

The present invention is described in further detail in connection with the following examples which illustrate or simulate various aspects involved in the practice of the invention. It is to be understood that all changes that come within the spirit of the invention are desired to be protected and thus the invention is not to be construed as limited by these examples.

### EXAMPLES

Mimicking the *in vivo* process, the application of an external electrical stimulation to accelerating wound healing was explored. Well aligned silk fibers (e.g., spider or silkworm), prepared from electrospinning, served as a cell culture scaffold due to its superior mechanical properties and biocompatibility. The electro-spun (E-spun) fibers mimicked the locally oriented ECM proteins in native tissue to facilitate fibroblast cell polarization and activation. Incorporating a low dose of oxidized single-wall carbon nanotubes (SWCNTs) into fibrous proteins such as collagen and silk can not only diminish the toxicity of SWCNTs but also effectively modulate stem cell differentiation to neural cells. CNT was added to silk to achieve electrically conductive protein fibers. A low voltage was adequate to deliver a square-wave pulse potential to the diabetic fibroblasts cells grown on well-aligned silk-CNT composite fibers to achieve renewed collagen and MMP synthesis serving for chronic wound healing and tissue regeneration

### Transformed diabetic dermal fibroblast cells to accelerate wound healing

In this experiment, diabetic dermal fibroblast cells were transformed by silk-CNT mediated electrical stimulation. Cells seeded on free-standing silk-CNT fibers polarized along the fiber direction in hours. The silk-CNT fibers were electrical conductive, stable, biocompatible and degradable in long term. When an electric field was applied for 6 h in the direction of fiber alignment, the cells were stimulated and were found to synthesize COLI and COLIII 5 and 20 folds higher, respectively, resulting in a COLI/COLIII ratio of 0.46. The synthesis of MMPs was also suppressed. This can be important, as it warrants a proper balance between collagen synthesis and degradation to allow the granulation tissue building up at the wound area for proper healing. Even after reseeded on a plain plastic substrate for 5 days, the cells continued to produce collagen at a significantly high level, indicating the potential of cell transplant.

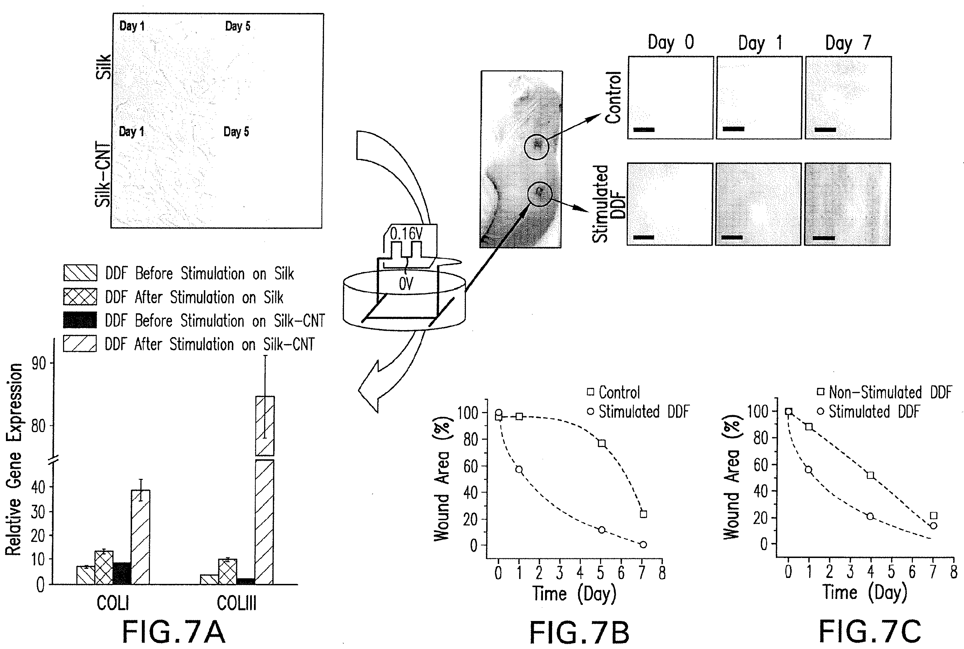

A wound healing experiment was also performed on a female nude mouse of over 40 weeks old. Two wounds were created: one left without treatment and the other was locally treated with transformed diabetic fibroblast cells for 12 h. As shown in Fig. 7, wound healing was markedly accelerated by transferring the cells to the local wound region, and the difference was the most significant by Day 1 (the wound was more than 50% smaller than the control). By Day 7, the wound was completely healed with the transformed cells and no scar was formed. The wound left blank gradually healed, however scar was formed on Day 9.

### Materials

Spider silk proteins or silkworm silk fibroin were dissolved in 1,1,1,3,3,3-hexafluoro-2-propanol (HFIP) for electrospinning.

High purity single-walled carbon nanotubes (SWCNTs) were oxidized by sonicating in 8 M H₂SO₄ and 8 M HNO₃ solutions at 70 °C for 3 hrs to achieve surface functionalization with hydrophilic carboxyl groups. The oxidized SWCNT suspension was then centrifuged at 1398 g force for 10 min followed by filtration with 0.22 µm isopore membrane and re-dispersed in HFIP. 0.05%, 0.5% or 1.0% (w/v) of the oxidized SWCNT was added to silk protein solution for electrospinning to generate silk-CNT composite fibers.

### Electrospinning of Free-standing Silk and Silk-CNT Fibers

Aligned freestanding silk and silk-CNT fibers were prepared using a home-built electrospinning system. A syringe pump was powered by a high-voltage power supply to deliver a voltage of 25 kV to the tip of a blunt needle. A protein solution of 100 mg/mL was ejected at a flow rate of 0.6 ml/min for 30 s to produce continuous, high-density fibers that were collected on two parallel metal plates, which were grounded and placed 10 mm apart and 15 cm below the needle. Due to the electrostatic interactions, the E-spun fibers were well-aligned and stretched across the two plates. Free-standing fibers were collected for mechanical tests. For microscopic characterization and cell culture, fibers were transferred onto plastic substrates pre-cut from a Petri dish or onto thin silicon gels.

### Cell Culture

Frozen primary human diabetic dermal fibroblast (DDF) cells, isolated from patients with type II diabetes immediately following amputation, and non-diabetic dermal fibroblast (NDF) cell controls post-mortem, were thawed then cultured in T-75 tissue culture flask (Corning, Manassas, VA) with Dulbecco's Modified Eagle Medium (DMEM) supplemented with 10% fetal bovine serum (Corning, Manassas, VA), 1% MEM Non-essential Amino Acid (Gibco, Carlsbad, CA), 1% MEM Vitamin (Gibco, Carlsbad, CA) and 2% Penicillin-Streptomycin (10,000 U/mL) (Gibco, Carlsbad, CA) and incubated at 37_{°}C with 5% CO₂. The medium was changed every other day and the cells were passaged every 3 days. At confluence, cells were detached with Trypsin-EDTA (0.25%) (Gibco, Carlsbad, CA) and plated on target matrices with a seeding density of 8000 cells/cm² for various studies.

### System for Electrical Stimulation of Cells

A home-built portable electrical stimulation system was employed to stimulate the fibroblast cells. The system was powered by a dual 9 V battery module. Two pieces of gold wires of 1 mm thick and 1 cm long were placed parallel in cell culture medium and set 10 mm apart to create an electrical field. After cells were seeded on the E-spun fiber matrices and cultured for 12 h, they were placed in the electric field with fibers aligned along the field. A voltage of 0.16 V at a frequency of 60 Hz was applied for a desired period of time. Ten-minute interval was added between every 20 minutes stimulation to discharge the electrodes to avoid protein aggregation.

### In vivo Wound Healing Assay

Immuno-deficient nude mice of 8-month old were used in the study. After anaesthetization, two 4 mm × 4 mm skin excisional wounds of 4 mm deep were created in the middle of the dorsum. Thin silicone gel substrates (made from SYLGARD^{®} 184, Dow Corning Midland, MI) were used to support aligned silk-CNT fibers. DDF cells were seeded on the fibers at a density of 20000 cells/cm² and cultured for 12 h before they were stimulated for 6 hours under the same condition as that in *in vitro* experiment. For one mouse, one of the two wounds was patched by the silicone gel with electrically stimulated cells, and the other wound was untreated as a control. For the second mouse, the two wounds were patched by silicone gels with electrically stimulated cells and unstimulated cells, respectively. Mice were caged individually after fully recovered from anesthesia. Silicone gel substrates were removed 24 hours later. Wound healing was monitored daily.

### Protein expression in dermal fibroblast cells from normal and diabetic individuals

To examine the functionality of DDF and NDF cells in collagen synthesis, immunostaining of these cells against COLI and COLIII was carried out. Evidently, COLIII expression was lower in DDF cells than in NDF cells (0.58:1), while COLI expression was higher in DDF cells than in NDF cells (1.48:1). Overall, the COLI/COLIII ratio was 3.95 in DDF cells in comparison to that of 1.55 in NDF cells, however the total amount of collagen was similar in the two cell types. mRNA level analysis was also performed, and the result was consistent with that of protein level analysis. Less COLIII and excess COLI typically forms stiff and thick hybrid fibers. At the tissue growth stage of wound healing, soft, flexible COLIII can be more desirable than COLI for the formation of granulation tissue to cover up the wound bed. The high COLI/COLIII ratio in DDF cells indicates these cells are incapable of supplying proteins to constitute granulation tissues. The expression levels of MMP-2 and MMP-9, the key enzymes that degrade collagen in dermal tissue. The result of RT-qPCR analysis indicates that their expression levels in DDF cells were 5.3 and 2.5 times higher than in NDF cells, respectively. The overexpression of MMP inevitably increased the degradation of newly synthesized collagen molecules before they were proper assembled and deposited to form new tissues. This is consistent with reports that MMP2 and MMP9 are overly expressed in diabetes patients. The aberrant expression of collagen and MMPs is one of the key factors contributing to the wound healing deficiency of diabetes patients.

### Characterization of silk and silk-CNT matrices

Silk and silk-CNT (0.05% wt.) fibers were prepared by E-spinning. These fibers were unidirectionally aligned, mimicking the local alignment of matrix proteins in native ECM. Fiber alignment was analyzed using 2-D fast Fourier transform (2-D FFT) approach to process 2048×2048 pixels of 8-bit gray-scale images using ImageJ software supported by an oval profile plug-in. Alignment index was defined by the ratio of the highest and the lowest peak value in a FFT plot. The alignment index of silk-CNT fibers (0.36 ± 0.03) is much higher than that of silk (0.09 ± 0.03), suggesting a better alignment of silk-CNT fibers than silk fibers (Table 1). Mechanical test was performed by stretching the fibers along the direction of fiber alignment. Young's modulus was derived from the load vs. stretching displacement curves.

Silk-CNT fibers were found to be 1.6 times more rigid than silk fibers. According to high-resolution AFM images, the silk-CNT fibers are thinner than the silk fibers (Table 1). This is ascribed to the lower solution viscosity of silk-CNT than that of silk due to the presence of CNT as an impurity in silk solution. Taken together, the addition of CNT to silk resulted in stiffer, thinner, better aligned and higher density fibers. CNT also increased the fiber conductivity, evidenced by the decrease of fiber resistivity from 3.1×10⁴ Ω·m of pure silk to 6.4×10² Ω·m of silk-CNT along the direction of fiber alignment. Thus, silk-CNT fibers can transmit electrical signal more efficiently than silk fibers.

**Table 1. Physical properties of E-spun silk and silk-CNT fibers prepared from a protein solution containing 100 mg/mL silk with or without 0.05 w% CNT.**

| **Material** | **Alignment Index** | **Solution Viscosity (cP)** | **Young's Modulus (GPa)** | **Diameter(µm)** | **Mean Resistivity (Ω·m)** |
|---|---|---|---|---|---|
| Silk | 0.09±0.03 | 17.1±0.1 | 3.9±0.2 | 1.4±0.1 | 3.1×10 |
| Silk CNT | 0.36±0.03 | 14. | 6±0.2 6.2±0.2 | 1.1±0.2 | 6.4×0 |

### Cell Polarization and Matrix Biocompatibility

Unidirectionally aligned fibers provide physical cues to induce cell polarization. DDF and NDF cells grown on silk and silk-CNT E-spun fibers were measured at day 1 and day 5. Cell length and width were measured from images. At day 1, the length-to-width ratio of DDF cells on silk and silk-CNT fibers was 3.6±0.3 and 3.7±0.2, respectively, and that of NDF cells was 2.8±0.1 µm and 4.7±0.3 µm, respectively. At day 5, the length-to-width ratio of DDF cells reached 4.2±0.4 and 7.0±0.4 on silk and silk-CNT fibers, and those of NDF cells were 4.8±0.3 and 7.1±0.5. While no significant difference of cell polarization was found between diabetic and normal dermal fibroblast cells (p > 0.05), cells were more polarized on silk-CNT fibers than on silk fibers (p <0.01) with increased culture time. In contrast, cells grown on flat plastic or gelatin substrates retained the spindle-shape and were less polarized over time. The enhanced polarization of cells on silk-CNT is likely due to the increased stiffness of aligned matrix that promotes cytoskeleton stretching and stimulates cell contraction to augment collagen production.

To examine biocompatibility of the matrices, proliferation and viability tests were carried out, with DDF or NDF cells cultured on plastic Petri dish and gelatin coated Petri dish as controls for a hard and a soft substrate, respectively. While CNT can increase the conductivity of silk fibers, excess amount of CNT is known to cause cytotoxicity. Therefore, different amount of CNT was incorporated into silk to determine the optimum CNT amount in the composite fibers. Initially, cells on gelatin, plastic, silk and silk-CNT fibers proliferated at a similar rate. After Day 2, cells on silk and silk-CNT fibers grew slower than those on plastic and gelatin. Cells on both gelatin and plastic reached confluence by Day 3 according to the plateaued proliferation curves. This was also confirmed by optical images of cells grown on the dishes. The curves for cells on silk and silk-CNT fibers were also plateaued but at a much lower absorbance. This is ascribed to fiber induced cell polarization that increases cell mobility while decreases the rate of cell proliferation. While the proliferation profiles of cells grown on fibers containing 0.05% to 0.5% CNT were similar, an apparent drop was observed for cells on 0.1% CNT. For comparison, cells were grown on plastic substrates coated with homogeneous silk and silk-CNT films. While the shape of cells on the smooth films was similar to that on plastic and gelatin, the cells proliferated much slower. Cell viability was tested after 24 hours cell culture on various matrices. The viability of cells on silk film, silk fibers and silk fibers containing 0.05% CNT was equally high (>95%), but decreased with the increase of CNT percentage in silk-CNT fibers. The viability of cells on silk-CNT films was as low as 84.3-85.3% for DDF and 81.0-85.3% for NDF. The silk-CNT film was unstable in cell culture medium. When it fell apart in the medium, CNT became in direct contact with cells. It is known that excess amount of free CNT uptake by cells can trigger the release of digestive enzymes, causing the damage of mitochondrial membrane and leading to reduced cell survival rate. When silk-CNT mixture was spun into fibers, due to the unique features of CNT, CNTs preferentially distributed at the interior of a fiber and aligned along the fiber axis (see Discussion section). At a low CNT concentration, CNTs were buried in silk proteins and was not exposed to cells, giving rise to high cell viability. At high CNT concentrations, excess CNTs might be present on the surface of the fibers and directly interact with cells giving rise to reduced cell survival rate. With the increase of CNT concentration from 0% to 0.05%, 0.5% and 1%, cell viability on silk-CNT fibers changed from 96.4% to 95.6%, 93.3% and 86.3% for DDF, and 93.1% to 91.7%, 90.2% and 85.5% for NDF. Noticeably, silk fibers containing 0.05% and 0.5% CNT resulted in the cell viability comparable to that on pure silk.

Both DDF and NDF cells demonstrated similar responses to various matrices and no significant difference was observed. Taken together, cells on silk-CNT films proliferated slowly with a low viability; cells on silk-CNT fibers proliferated at a medium rate with a higher viability at a lower CNT percentage. Silk fibers containing 0.05% CNT was chosen in the remaining experiments due to its excellent biocompatibility.

### Effect of matrix on fibroblasts for collagen synthesis

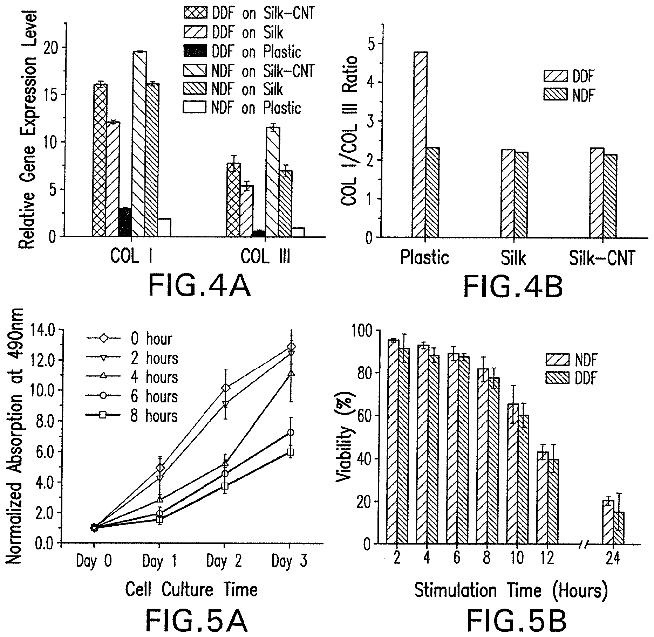

RT-qPCR analysis was carried out to quantitatively evaluate the level of COLI and COLIII synthesis by DDF and NDF cells, which were cultured for 5 days on silk-CNT fibers, silk fibers and plastic Petri dish, respectively. As shown in Fig. 4A, fibroblast cells on all matrices expressed COLI at a higher level than COLIII. Cells on silk and silk-CNT expressed collagen at a much higher level than cells on plastic, and the elevation is more significant in NDF cells than in DDF cells. It is also evident that cells on silk-CNT expressed collagen at a higher level than those on silk. As aforementioned, both DDF and NDF cells were more polarized on silk-CNT than on silk fibers, and well-polarized fibroblast cells on aligned, stiff fibers could produce collagen at a higher level. Note that the protein expression for cells on gelatin (not shown) is very similar to that on plastic. It suggests that matrix stiffness alone doesn't regulate collagen synthesis by the fibroblast cells.

COLI/COLIII ratio was calculated and compared for NDF and DDF cells cultured on various matrices. As shown in Fig. 4B, the ratio varied in a narrow range (2.12 to 2.34) for NDF cells cultured on all three types of matrices. In contrast, while the COLI/COLIII ratio was 4.76 for DDF cells on plastic Petri dish, it reduced to 2.27 and 2.32, respectively, after the DDF cells were cultured on silk and silk-CNT fibers. Thus, silk and silk-CNT matrices promoted COLI and COLIII synthesis by fibroblast cells and renewed the abnormal COLI/COLIII ratio in DDF cells while retained the normal COLI/COLIII ratio in NDF cells.

### Cell Responses to Electrical Stimulation

Due to the increased conductivity of silk-CNT fibers, the electrical stimulation of the fibroblast cells was explored by applying an electric potential along the fibers. To optimize the stimulation time while retain cell survival, the dependence of cell proliferation and viability on the stimulation time was examined. As shown in Fig. 5A, cell proliferation rate increased when the stimulation time increased from 0 h to 6 h. 8 h stimulation didn't further increase the proliferation rate. While the data was generated using DDF cells, NDF cells showed very similar proliferation profile. With cells without stimulation as a control, cell viability after the cells were stimulated for 2-24 h was examined and subsequently cultured on the same matrix for 24 h. Both DDF and NDF cells responded similarly. Apparently, electrical stimulation accelerated cell growth, however, a longer than 6 h stimulation affected cell viability. Considering both factors, 6 h electrical stimulation was chosen to carry out the rest of the experiments.

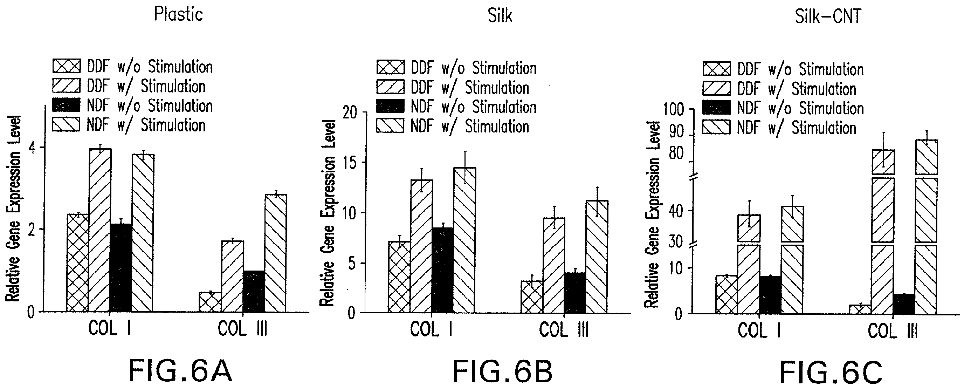

RT-qPCR analysis was performed to quantitatively compare collagen synthesis by NDF and DDF cells cultured on silk-CNT fibers, silk fibers, and plastic Petri dish for 12 h before 6 h electrical stimulation (Fig. 6). The expression levels were normalized towards COLIII expression of NDF cells cultured on plastic Petri dish before stimulation (set to 1). The effect of electrical stimulation is three folds: (1) it boosted COLI and COLIII synthesis for both NDF and DDF cells on all three matrices; (2) COLIII production was enhanced more significantly than COLI; (3) dramatic enhancement was seen in cells on silk-CNT, leading to 5.2-fold and 23.7-fold increase of COLI and COLIII production for DDF cells, and 5.1-fold and 21.4-fold increase of COLI and COLIII production for NDF cells. Additionally, upon electrical stimulation of silk-CNT, COLI/COLIII ratio was decreased from 1.96 to 0.47 for NDF cells, and decreased from 4.49 to 0.46 for DDF cells. Thus, the electrically conductive silk-CNT fibers can effectively transmit electrical signals and stimulate the cells to synthesize significantly higher amount of COLI and COLIII. The stimulation induced remarkably high COLIII expression in DDF cells, signifies potential application in treating chronic wound of diabetes patients.

It can be critical that the renewed cells can retain the function for a prolonged period of time after the termination of stimulation. This was studied by transferring the stimulated cells to plastic Petri dishes and performed RT-qPCR analysis at days 0, 1, 3 and 5 of culture to monitor the change of COLI, COLIII, MMP2 and MMP9 levels with time. The expression levels of both MMP2 and MMP9 were higher in DDF cells than in NDF cells. Right after electrical stimulation, the relative expression levels of MMP2 and MMP9 dropped by 3.3 and 1.6 folds, respectively, in DDF cells, whereas they remained the same in NDF cells. From Day 1 to Day 5, the MMP expression levels slightly increased but in the normal range for both cell types. For COLI and COLIII, after the drastic increase of their levels of expression right after stimulation, the expression levels gradually dropped over time. Nevertheless, COLI and COLIII expression remained significantly higher than those before stimulation. For instance, the expressions of COLI and COLIII remained 2.7 times and 18.1 times higher for DDF cells by day 5, and were 1.6 times and 13.6 times higher for NDF cells by day 5. Importantly, COLIII expression stayed higher than COLI. The result implies that the stimulation effect can last for a period of time even after the cells were transferred to a completely different substrate.

An *in vitro* biodegradation test was carried out to evaluate the stability of silk-CNT fibers as cell culture matrix. High concentration collagenase complex (100 U/ml) was applied for various time periods to mimic the high enzyme activity during inflammation process of wound healing. Silk-CNT degraded faster than silk. The weight loss of the E-spun silk-CNT fibers was 48.2% by Day 14, 85.3 % by Day 25 and 100% by Day 35. Thus, silk-CNT fibers are stable for at least 2 weeks even with the presence of high concentration of enzymes, and they are degradable after long-term enzyme treatment.

### In Vivo Wound Healing Assay

An *in vivo* wound healing assay was carried out to evaluate the effect of the electrically stimulated DDF cells on wound healing. A significant difference in wound healing kinetics was observed for untreated wound (control) and electrically stimulated DDF treated wound, both wounds were created identically on the same mouse. As shown in Fig. 7A, after 24 h cell treatment, the wound area reduced by 42% in comparison to 8% reduction for the untreated wound. Following the treatment, the wound healing process was further monitored. It was found that the wound area reduced by 90% by day 5 and the wound was completely healed by day 7. In contrast, the area of untreated wound reduced by 50% by day 5 and by 80% by day 7. Fig. 7B illustrates the kinetic change of wound area with and without cell treatment. The result suggests that the electrically stimulated DDF cells greatly accelerated the process of wound healing, and the effect was long lasting.

According to testing above, aligned matrix alone can also stimulate the DDF cells to produce more collagen. However, it was less effective than electrical stimulation. To reveal the impact of electrical stimulation, another experiment was performed on a different mouse to compare the effect of unstimulated DDF cells and electrically stimulated DDF cells, both types of cells were pre-cultured on aligned silk-CNT fibers for 12 h (Fig. 7C). Consistent with the result of *in vitro* studies, aligned silk-CNT matrix also promoted wound healing, and electrical stimulation had a greater impact.

### Discussion

The transformation of DDF cells was achieved by electrical stimulation of the cells on aligned silk-CNT matrix to tackle the challenge of wound healing in diabetes patients. The transformed cells overcome the deficiencies of the original cells' low COLIII expression and high MMP expression that limit the supply of a large amount of COLIII for the formation of granulation tissues at the wound of diabetes patients, prohibiting wound healing and leading to chronic wounds. The *in vitro* and *in vivo* studies demonstrated that the transformed cells effectively accelerate a wound healing process.

Freestanding tissue engineering scaffolds are highly desirable for *in vitro* and *in vivo* applications. They are required to be sufficiently flexible, tough, stable and biocompatible for effortless handling and for adequate support of cell development. Silk-CNT fibers generated in this study meet such criteria. While a high percentage of CNT is harmful to cells, silk fibers with 0.05% CNT are not only biocompatible but also adequately conductive to transmit electrical signals to stimulate the cells (see discussions below). CNTs in the silk-CNT fiber likely align in the direction of the fibers attributed to the applied electric field during E-spinning as well as CNT's conductivity, rigidity and one-dimensional nanostructure. Due to its large surface area and hydrophobic nature, CNTs are presumably surrounded by silk proteins, which are rich in glycine and alanine residues, granting strong silk-CNT interaction and well integrated composite fibers. This effectively prevented the direct contact of cells with CNT, which can cause cell damage and reduce cell viability.

Incorporation of CNT to silk fibers enhanced dermal fibroblast cell polarization on the matrix, consistent with our previous observation. The stiffer, well-aligned silk-CNT fiber substrates promoted the cells to synthesize an increased amount of COLIII than COLI and reduce the COLI/COLIII ratio, leading to a softer extracellular matrix. Impressively, the application of electrical stimulation induced a dramatic augmentation of collagen production in cells grown on aligned silk-CNT fibers in contrast to the slight change in cells on silk fibers, which lacks electrical conductivity. It resulted in a 5-fold increase of COLI production and a 45-fold increase of COLIII production, giving rise to a COLI/COLIII ratio of 0.46 in both DDF and NDF cells. The lower-than-normal COLI/COLIII ratio is desirable should the cells be resupplied to the patient for remodeling the patients' stiff tissue to the normal level. Electrical stimulation likely boosted the synthesis of FGF that promotes the transition of fibroblast to myofibroblast, which is the active form of the cell for protein synthesis. The supply of a great amount of collagen with low COLI/COLIII ratio is desirable for forming granulation tissues during wound healing. Strikingly, electrical stimulation also suppressed the synthesis of MMPs in DDF cells. This provides a proper balance between collagen synthesis and degradation to allow the granulation tissue building up at the wound area, in oppose to the overexpression of MMPs in DDF cells that results in the acceleration of matrix protein degradation leading to chronic wounds. The effectiveness of the transformed DDF cells to accelerate wound healing was confirmed in a mouse model.

In embodiments of this invention, instead of relying on cells from matching donors, dermal fibroblast cells of a diabetes patient can be extracted and renewed *ex vivo,* then injected back to the patient at the wound area for treatment without the concern of immune-rejection. The transformed DDF cells were reseeded on a plain plastic substrate and grew for 5 days, the cells remained to produce collagen at a significantly high level. The transformed DDF cells should be able to retain the functionality when they are transferred to the matrix at the wound region, and they have a long lasting effect on remodeling the tissue to accelerate wound healing, as demonstrated in the animal study (Fig. 7). In the animal study, the cells were patched at the wound for 24 h only. Alternatively, the free-standing E-spun fibers with transformed cells can be implanted. The silk fibers with 0.05% CNT are biocompatible. In this case, patients can undergo a physical therapy by applying a mild potential locally to restimulate the cells periodically when needed. NDF cells can also be stimulated to produce significantly higher amount of collagen at a low COLI/COLIII ratio, the same treatment strategies can be applied to wound healing for non-diabetes patients.

In most of the acute wound healings, inflammation activity reduces after the first week of the wound and the granulation tissue formation starts as early as 10 hours.

In an acute wound healing process, inflammation activity is turned on soon after a wound is created, accompanied by the increased MMP expression level to clean the wound bed. For diabetes patients, MMP expression level in DDF cells is much higher. It requires the implant matrix to be stable to survive the high enzyme activity. It was shown that after 100 U/mL collagenase was applied, the silk-CNT fibers were degraded by 48% by day 14. This enzyme concentration is 100 times higher than that was used by, who reported a 50-75% degradation by day 12 for silk fibroin tissue engineering scaffolds. Thus, the silk-CNT fibers are very stable, however they are less stable than pure silk fibers. Additionally, the matrix retained the aligned fiber matrix, which provides support and guide cell polarization to retain the cell activity and grant the granulation tissue formation. Its degradation at a later time is equally critical to avoid a secondary damage caused by the implant material. The silk-CNT fibers demonstrated adequate stability and degradability to support wound healing.

### Electrospun Protein-CNT Composite Fibers in Stimulation of Fibroblast from Pelvic Organ Prolapse (POP) Patients

In this example, varied percentage of CNT was incorporated into collagen and silk to generate protein-CNT electro-spun (E-spun) fibers. Comparative study was carried out to unravel the distinctive effect of CNT on collagen and silk fibers in fiber structure, mechanics, electrical conductivity, and in mediating fibroblast stimulation for pelvic floor connective tissue repair.

Fibroblasts derived from the tissue of a pelvic organ prolapse (POP) patient were used as a model system. These cells are characterized by reduced collagen productivity and an abnormally high collagen types I (COLI) to III (COLIII) ratio of the synthesized collagen. Fibrillar COLI offers high tensile strength to a tissue matrix, whereas COLIII is found alongside COLI in tissues that require flexibility and distension. The collagen disorder in POP gives rise to a loose and fragile collagen fiber network that fails to properly integrate with other tissue constituents, and consequently, reduces the strength of the connective tissue leading to failure in supporting the pelvic organs. The protein-CNT fibers provided unique structural and mechanical cues to regulate the fibroblasts producing collagen at a reduced COLI/COLIII ratio; due to the high conductivity, silk-CNT fibers effectively mediated ES of the cells to boost collagen synthesis. It offers a simple, direct and effective way to restore the function of patient cells, which can be potentially used for personalized cell therapeutic treatment to achieve tissue repair.

### Materials and Methods

Dragline spider silk proteins and collagen type I of calf skin were dissolved in 1,1,1,3,3,3-hexafluoro-2-propanol (HFIP) for electrospinning. High purity single-walled CNTs were oxidized following the protocol in previous reports. They were added to collagen or silk solution of 100 mg/mL at 0, 0.05%, 0.25% or 0.5% (w/v) to generate E-spun protein-CNT composite fibers.

### Fabrication and characterization of E-spun fibers.

Aligned, freestanding protein and protein-CNT fibers were prepared using a home-built electrospinning system following the protocol in B. Zhu et al., Optimization of Glutaraldehyde Vapor Treatment for Electrospun Collagen/Silk Tissue Engineering Scaffolds, ACS omega 2 (2017) 2439-2450, herein incorporated by reference. The fibers were transferred onto glass slides for microscopic characterization and cell culture. Prior to any test, the fibers were sterilized/post-treated by 70% ethanol followed by 1 h UV irradiation.

Fiber alignment was analyzed using the 2-D fast Fourier transform (2-D FFT) approach to process gray-scale images with an oval profile plug-in. The alignment index was defined by the ratio of the highest and the lowest peak values in a FFT plot. Fiber diameter was measured in AFM images collected by an atomic force microscope. Fiber resistivity was derived from current-voltage curves of fibers aligned between two pure gold electrodes using a patch clamp amplifier. Load vs. stretching displacement curves were collected to derive the ultimate tensile strength, ultimate strain and Young's modulus for each fiber type based on more than 1000 fibers from 20 samples for statistical analysis.

### Cell analysis and electrical stimulation.

Vaginal fibroblast, extracted from the biopsy harvested at the posterior vaginal fornix of a POP patient, was used as a cell model in this study. Cells were seeded at a density of 8000 cells/cm² on various matrices. Cell viability test was carried out by the CellTiter 96^{®} Aqueous One Solution Cell Proliferation Assay (MTS) following the manufacturer's protocol, and examined at 24 h post-plating. Cell polarity was characterized by the cell length-to-width ratio, which was evaluated based on optical images. The effective cell width was determined using the ratio of cell area to cell length.

ES was conducted by applying 0.16 V at 60 Hz to cells pre-seeded on the protein fibers that were placed between two parallel gold electrodes (10 mm apart). 6 h ES was chosen based on cell viability analysis.

### RT-qPCR analysis.

Total RNA was extracted from cells cultured on various matrices, and reverse-transcription was carried out by a SuperScript^{®} III kit (Invitrogen, Carlsbad, CA). RT-qPCR was performed using an ABI Prism 700 (Applied Biosystem, Foster City, CA) with TaqMan^{®} Gene Expression Master Mix and TaqMan^{®} Gene Expression Assay (Life Technology, Madison, WI). Log 2 fold change was derived based on Cₜ calculation using GAPDH as a house-keeping gene and relative to COL3A1 expression in cells cultured on plastic in the absence of ES. Data analysis was carried out using the 2^{-ΔΔCT} method for relative quantification based on five replicate measurements.

### Statistical analysis.

Fiber alignment index, fiber resistivity, cell viability, and gene expression data were analyzed by one-way ANOVA, and individual differences were determined by Tukey's multiple comparison test. Collagen expression level on various matrices with and without ES was analyzed by 2-way ANOVA (matrices*ES) with Tukey's multiple comparison test using the statistical software package SPSS (version 22; IBM, Armonk, NY).

### Results

To achieve mechanically strong, well-aligned thin fibers mimicking protein fibers in a native tissue, we chose the protein concentration of 100 mg/mL for both collagen and silk and the optimized electrospinning conditions as concluded from a previous study. The effects of CNT addition were investigated.

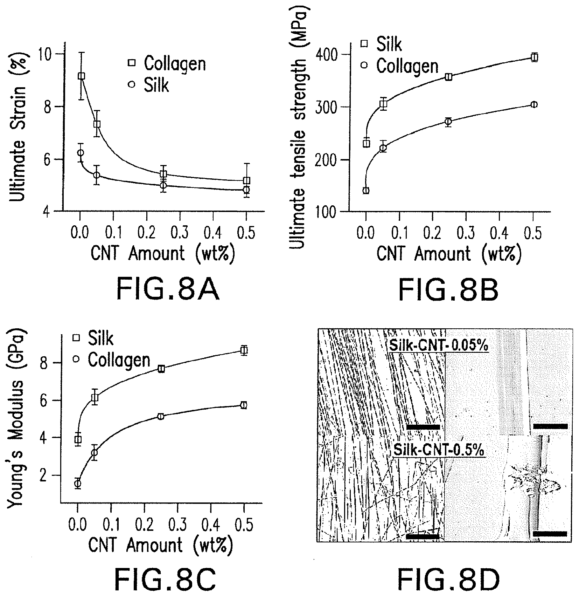

The mechanical properties of E-spun fibers were characterized by stress-strain tests (Fig. 8). Compared to pure collagen fibers, pure silk fibers demonstrated higher ultimate tensile strength and Young's modulus but lower ultimate strain. It suggests that silk fibers are stronger whereas collagen fibers are more stretchable. Due to CNT's high tensile strength, high stiffness and rigidity, the addition of CNT enhanced the fiber strength and stiffness for both collagen and silk but in the expense of sacrificing the extensibility. Fiber strength and stiffness were further increased with the increase of CNT percentage but at a much lower rate, whereas the ultimate strain decreased and was plateaued at higher CNT percentages. It is surmised that only a limited amount of CNT can integrate with the proteins effectively to improve fiber strength. Noticeably, the decrease of ultimate strain with CNT addition was more significant in COL-CNT fibers than in silk-CNT fibers due to distinctive interactions between collagen-CNT and silk-CNT (See Discussion).

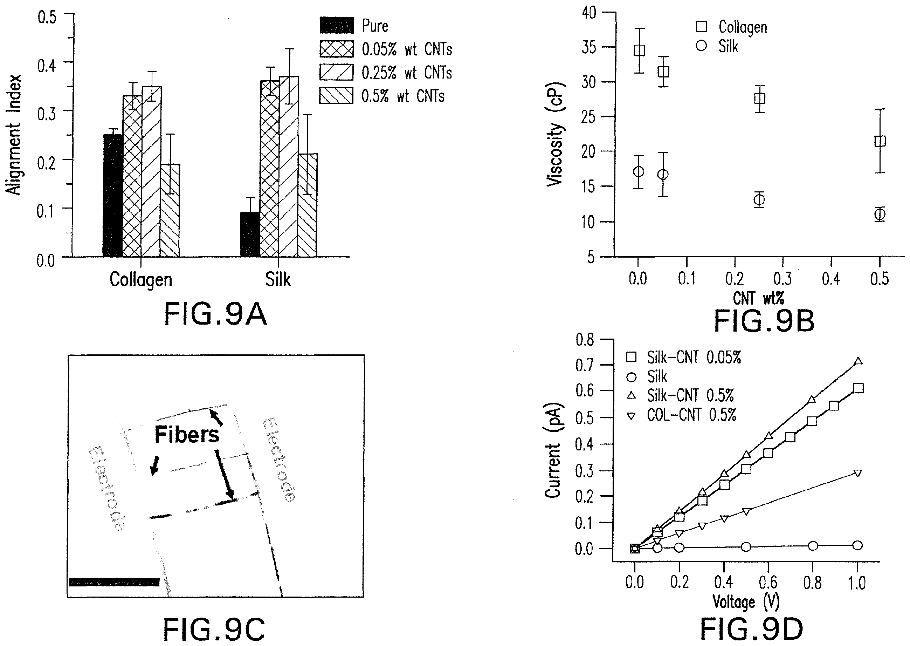

Fig. 9A illustrates the variation of fiber alignment index with the fiber types. The addition of a 0.05% CNT improved the fiber alignment significantly, particularly for silk fibers. Further increase of CNT to 0.25% improve the alignment slightly, and a 0.5% CNT resulted in a decrease of fiber alignment. It is known that the viscosity of a solution, associated with protein concentration and solution composition, plays an important role in fiber alignment. Consistently, given the same protein concentration (100 mg/mL), a pure collagen or silk solution had a viscosity of 34.5 cP or 17.1 cP and produced fibers with an alignment index of 0.25 or 0.09. Because CNT is present as an insoluble impurity, the addition of increased CNT led to a decrease of solution viscosity (Fig. 9B), and is expected to decrease fiber alignment. The initial increase of fiber alignment with CNT addition suggests that other factors than solution viscosity may play a more significant role in fiber alignment. The addition of CNT increased fiber stiffness and conductivity, both positively impact the fiber alignment. The interplay of these opposing effects determined the optimum CNT percentage to achieve well-aligned protein fibers (Fig. 9A).

Current-voltage curves were collected from aligned fibers (Figs. 9C-D), and the resistivity was derived from the slop of the linear curves. It is evident that the addition of CNT effectively increased the fiber conductivity. In the case of collagen, the resistivity of pure collagen and COL-CNT-0.05% fibers was too high to detect using the current patch clamp amplifier. The resistivity of COL-CNT-0.5% fibers was 3273 Ω·m, 5 times higher than the resistivity of silk-CNT-0.05% fibers (645 Ω·m). Note that when the CNT percentage was higher than 0.5%, COL-CNT fibers were largely misaligned and are unfavorable; the silk-CNT fibers became unachievable due to the extremely low solution viscosity.

Fibers with high alignment index, medium stiffness, high conductivity, ~1.0 µm in diameter (mimicking the dimension of native fibers) and minimum CNT addition were found to be desirable to serve as a matrix for cell culture. Considering all these factors, silk-CNT-0.05% fiber was determined as the best candidate. Though silk-CNT-0.25% showed similar properties as silk-CNT-0.05%, a 5 times higher dosage of CNT is undesirable due to the increased risk of cytotoxicity. The nearly 20% reduction of fiber diameter is also an obstacle concerning the effective interaction between cells and fibers to regulate cell function. Among the COL-CNT fibers, COL-CNT-0.05% and COL-CNT-0.25% fibers are better aligned, but are thicker and weaker than COL-CNT-0.5% fibers. Additionally, they lack electrical conductivity. Thus, COL-CNT-0.5% fibers were included in cell studies.

An excess amount of free CNT uptaken by cells can trigger the release of digestive enzymes, causing the damage of mitochondrial membrane and leading to reduced cell survival rate. The presence of 0.1% to 0.7% CNT in COL-CNT fibers had been found to not affect cell viability. In this example, the viability of cells grown on silk-CNT fibers containing 0%, 0.05% and 0.5% CNT was 97.1%, 97.3%, 94.1%, respectively. Statistical analysis indicates that the reduced cell viability on silk-CNT-0.5% fibers was statistically significant (p<0.05).

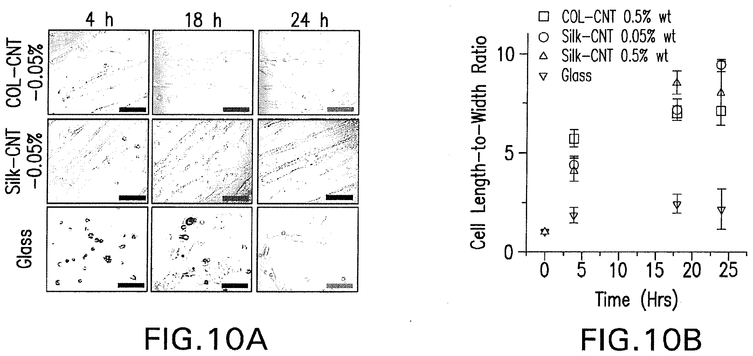

An aligned stiff matrix is known to induce cell polarization. As shown in Fig.10, at 4 h post-plating, cells on E-spun fibers have started to elongate along the fibers, in contrast to random cells on glass substrates. The fibroblasts were less polarized on silk-CNT than on COL-CNT. Overtime, the cells were further polarized, and became more polarized on silk-CNT than on COL-CNT. This is mainly because collagen fibers are more adhesive to cells than silk fibers. Consequently, cells can interact with the COL-CNT fibers faster and start the elongation earlier (at 4 h). Once the cells were attached, silk-CNT fibers promoted more efficient cell elongation than COL-CNT fibers as the silk-CNT fibers are stiffer and aligned better. It was also observed that cells on COL-CNT-0.5% and silk-CNT-0.5% fibers stopped further polarization at 18 h whereas cells on silk-CNT-0.05% fibers polarized further. This is ascribed to the mechanical strength and superior alignment of the silk-CNT-0.05% fibers that enabled constant support of fibroblast polarization in long-range overtime.

To investigate the modulation effect of the E-spun fibers, cells were seeded on fibers, cultured for 24 hours, then subjected to ES by applying an electric field in the direction of fiber alignment. Comparative study was carried out to examine COL1A1 and COL3A1 gene expression in cells on various matrices in the presence and absence of ES.

Compared to cells grown on pure protein fibers in the absence of ES, cells on the stiffer and better aligned protein-CNT fibers expressed COLIII at a significantly higher level (p<0.0001, n=9) and expressed COLI at a similar level (p>0.05). This led to a reduced COLI/COLIII ratio of the synthesized collagen, which can effectively remodel the matrix by reducing the matrix stiffness. ES demonstrated a dramatic effect on boosting the collagen productivity of cells grown on conductive fibers. For instance, ES boosted COLI and COLIII expressions by 20.8 folds and 21.5 folds, respectively, in cells on silk-CNT-0.05% fibers, but by 1.3 folds and 1.5 folds only in cells on pure silk fibers. ES didn't induce any significant change in cells on non-conductive pure collagen fibers (p>0.05). Silk-CNT-0.05% and silk-CNT-0.5% fibers exhibiting similar resistivity displayed similar effect. It is worth noting that ES didn't induce a significant change of the COLI/COLIII ratio for cells grown on silk-CNT-0.05% and silk-CNT-0.5% (p>0.05), but induced a significant decrease of the ratio on COL-CNT-0.5%. It was speculated that the presence of COLI in the protein fibers imposed a constantly high local concentration of COLI to the cells, prompting the cells to continually synthesize more COLIII than COLI in order to remodel the matrix.

The ability of incorporating a minute amount of extraneous agents into protein fibers offers a simple, robust yet mild approach for modulating the properties of tissue engineering scaffolds while retaining their inherent biocompatibility. Due to CNT's conductivity, rigidity and one-dimensional nanostructure, when a protein-CNT mixture is spun into fibers, CNTs align along the fiber axis. With a large surface area and hydrophobic nature, CNTs preferentially interact with proteins' hydrophobic domains that are present in the interior of a protein fiber. The protein-CNT fibers are stabilized by hydrogen bonding and electrostatic interaction due to the carboxyl groups on the oxidized CNTs and the positively charged amino acids in collagen and silk proteins. While it was no surprise the conductivity of protein fibers increases with the added CNT, it was striking that a minute amount of CNT (0.05%) had increased the silk fiber conductivity by 48 folds, but the conductivity of COL-CNT-0.05% fibers remained extremely low.

Thus the invention provides a free-standing, unidirectionally aligned silk-CNT fiber matrix that is useful in tissue regeneration, improving collagen production with desirable expression profile, and/or wound healing. Application of an electric field stimulated the diabetic fibroblast cells on an aligned silk-CNT fiber matrix supplies improved collagen production with low COLI/COLIII ratio, which is favorable in forming granulation tissue during wound healing. The electrical stimulation also suppressed the cell synthesis of MMPs, which provides a proper balance between collagen synthesis and degradation to allow the granulation tissue building up at the wound area, in oppose to the overexpression of MMPs in diabetic fibroblast cells resulting in acceleration of matrix protein degradation leading to chronic wounds. The transformed cells were shown to accelerate wound healing in a mouse model. A similar effect was shown to restore the function of fibroblast in patients with health conditions associated with connective tissue disorder, such as pelvic organ prolapse.

The invention can be applied to revive the function of fibroblast cells of diabetes patients. Replenishing transformed autologous cells to diabetes patients can restore the matrix protein expression profile, which reconditions the microenvironment to support proper cell function leading to a long-lasting influence for effective wound healing. Fibroblast cells are relatively abundant, easy to harvest, culture, expansion and stimulation *in vitro,* making them an ideal and inexpensive cell source for cell therapeutic treatment for chronic wound of diabetes patients.
The invention illustratively disclosed herein suitably may be practiced in the absence of any element, part, step, component, or ingredient which is not specifically disclosed herein.

## Claims

1. A cell therapy composition for improving collagen production, ratios of collagen types, tissue engineering or wound healing, comprising a support matrix on which cells are supported, wherein the support matrix is formed of fibers, wherein the fibers include unidirectionally aligned composite silk-carbon nanotube fibers.

2. The cell therapy composition of Claim 1, wherein the fibers are electrospun fibers.

3. The cell therapy composition of Claim 1 or 2, wherein the composite silk-carbon nanotube fibers comprise 0.05 wt% to 0.5 wt% carbon nanotubes.

4. The cell therapy composition of Claim 1 or 2, wherein the cells are fibroblast or smooth muscle cells.

5. The cell therapy composition of Claim 1 or 2, further comprising an electrical stimulator in electric supply combination with the matrix of fibers.

6. The cell therapy compositionof Claim 1, wherein the composite silk-carbon nanotube fibers are supported on a silicone gel substrate.

7. A method of tissue engineering, improving collagen production and/or ratios of collagen types, the method comprising:
seeding cells on a support matrix formed of fibers, wherein the fibers include unidirectionally aligned composite silk-carbon nanotube fibers;
applying an electric current to the support matrix to stimulate the cells.

8. The method of Claim 7, wherein the cells are fibroblasts or smooth muscle cells.

9. The method of one of Claim 7 or 8, further comprising collecting cells from the stimulated cell culture to apply to a tissue.

## Patentansprüche

1. Zelltherapiezusammensetzung zur Verbesserung der Kollagenproduktion, der Verhältnisse von Kollagentypen, der Gewebezüchtung oder der Wundheilung, umfassend eine Trägermatrix, auf der Zellen getragen werden, wobei die Trägermatrix aus Fasern gebildet ist, wobei die Fasern unidirektional ausgerichtete Seiden-Kohlenstoffnanoröhren-Verbundfasern umfassen.

2. Die Zelltherapiezusammensetzung nach Anspruch 1, wobei die Fasern elektrogesponnene Fasern sind.

3. Die Zelltherapiezusammensetzung nach Anspruch 1 oder 2, wobei die Seiden-Kohlenstoffnanoröhren-Verbundfasern 0.05 Gew.-% bis 0.5 Gew.-% Kohlenstoffnanoröhren umfassen.

4. Die Zelltherapiezusammensetzung nach Anspruch 1 oder 2, wobei die Zellen Fibroblasten oder glatte Muskelzellen sind.

5. Die Zelltherapiezusammensetzung nach Anspruch 1 oder 2, ferner umfassend einen elektrischen Stimulator in elektrischer Versorgungskombination mit der Matrix aus Fasern.

6. Die Zelltherapiezusammensetzung nach Anspruch 1, wobei die Seiden-Kohlenstoffnanoröhren-Verbundfasern auf einem Silikongelsubstrat getragen werden.

7. Ein Verfahren zur Gewebezüchtung, das die Kollagenproduktion und/oder die Verhältnisse der Kollagentypen verbessert, wobei das Verfahren Folgendes umfasst:
Aussäen von Zellen auf einer Trägermatrix aus Fasern, wobei die Fasern unidirektional ausgerichtete Seiden-Kohlenstoffnanoröhren-Verbundfasern umfassen;
Anlegen eines elektrischen Stroms an die Trägermatrix zur Stimulierung der Zellen.

8. Das Verfahren nach Anspruch 7, wobei die Zellen Fibroblasten oder glatte Muskelzellen sind.

9. Das Verfahren nach einem der Ansprüche 7 oder 8, ferner umfassend das Sammeln von Zellen aus der stimulierten Zellkultur zum Auftragen auf ein Gewebe.

## Revendications

1. Composition de thérapie cellulaire pour améliorer la production de collagène, les ratios de types de collagène, l'ingénierie tissulaire ou la cicatrisation des plaies, comprenant une matrice de support sur laquelle les cellules sont supportées, dans laquelle la matrice de support est formée de fibres, dans laquelle les fibres comprennent des fibres composites soie-nanotubes de carbone alignées de manière unidirectionnelle.

2. La composition de thérapie cellulaire de la revendication 1, dans laquelle les fibres sont des fibres électrofilées.

3. La composition de thérapie cellulaire de la revendication 1 ou 2, dans laquelle les fibres composites soie-nanotubes de carbone comprennent de 0,05 % en poids à 0,5 % en poids de nanotubes de carbone.

4. La composition de thérapie cellulaire de la revendication 1 ou 2, dans laquelle les cellules sont des fibroblastes ou des cellules musculaires lisses.

5. La composition de thérapie cellulaire de la revendication 1 ou 2, comprenant en outre un stimulateur électrique en combinaison d'alimentation électrique avec la matrice de fibres.

6. La composition de thérapie cellulaire de la revendication 1, dans laquelle les fibres composites soie-nanotubes de carbone sont supportées par un substrat de gel de silicone.

7. Procédé d'ingénierie tissulaire, améliorant la production de collagène et/ou les ratios de types de collagène, le procédé comprenant :
ensemencement de cellules sur une matrice de support formée de fibres, dans laquelle les fibres comprennent des fibres composites soie-nanotubes de carbone alignées de manière unidirectionnelle ;
l'application d'un courant électrique à la matrice de support pour stimuler les cellules.

8. Le procédé de la revendication 7, dans lequel les cellules sont des fibroblastes ou des cellules musculaires lisses.

9. Le procédé de l'une des revendications 7 ou 8, comprenant en outre la collecte de cellules de la culture cellulaire stimulée pour les appliquer à un tissu.
